# EUROPEAN PATENT APPLICATION

(11) **EP 3 495 824 A1**
(43) Date of publication of application: **12.06.2019**
(21) Application number: 17836811.4
(22) Date of filing: 25.07.2017
(51) Int. Cl.: G01N 35/08, C12M 1/00, C12M 1/34, G01N 21/05, G01N 21/78, G01N 37/00, C12N 15/09

(54) **ANALYSIS CELL, ANALYSIS DEVICE, ANALYSIS EQUIPMENT, AND ANALYSIS SYSTEM**

(30) Priority: 03.08.2016 JP 2016153132
(71) Applicant: Riken, Wako-shi, Saitama 351-0198 (JP); Kabushiki Kaisha Dnaform, Kanagawa 230-0046 (JP)
(72) Inventor: YAMAGATA Yutaka, Wako-shi Saitama 351-0198 (JP); AOKI Hiroyoshi, Wako-shi Saitama 351-0198 (JP); USUI Kengo, Wako-shi Saitama 351-0198 (JP); TANAKA Yuki, Wako-shi Saitama 351-0198 (JP); MATSUYAMA Norihiro, Yokohama-shi Kanagawa 230-0046 (JP); MITA Kanji, Yokohama-shi Kanagawa 230-0046 (JP)
(74) Representative: J A Kemp
(86) International application number: PCT/JP2017/026825
(87) International publication number: WO 2018/025705

(57) **Abstract**

The present invention provides a tool that can analyze a target in a sample with simple operations and can be downsized, and an analysis method using the same. The analysis cell of the present invention includes: a main substrate: a sample inlet cover member; and a gas outlet cover member. The main substrate includes a flow path, an inlet for a sample, and a gas outlet, and the inlet and the gas outlet communicate with an outside. The inlet communicates with an upstream end portion of the flow path and the gas outlet communicates with a downstream end portion of the flow path. The flow path has a shape that expands from an upstream side toward a downstream side of the flow path. The sample inlet cover member is a liquid-tight member and can be fixed to the inlet when the sample inlet cover member is in use. The gas outlet cover member is a liquid-tight and gas-permeable member and can be fixed to the gas outlet when the gas outlet cover member is in use.

## Description

### TECHNICAL FIELD

The present invention relates to an analysis cell, an analysis device, an analysis apparatus, and an analysis system.

### BACKGROUND ART

In recent years, for an infectious disease caused by viruses, bacteria, or the like, a common test method is to detect a target gene as an infection source in a biological sample. The target gene generally is detected by pretreating a collected sample, performing nucleic acid amplification of the target gene in the pretreated sample using primers, and detecting the presence or absence or amount of the nucleic acid amplification. Since the detection of the target gene requires a plurality of steps as described above and also requires a dedicated device and the like, the detection of the target gene is performed in a medical institution such as a hospital or a specialized testing institution.

On the other hand, there are actual circumstances where detection at the individual level is required instead of the detection at the testing institution or the like, as described in the following. For example, if someone has symptoms of a cold, it is desirable from the viewpoint of preventing secondary infection to others if he/she can check whether he/she is infected with influenza viruses at home in advance. Among various infectious diseases, especially sexually transmitted diseases caused by HIV viruses and *Candida* are often discovered late because patients hesitate to be examined at hospitals. Accordingly, if these diseases can be tested at home, early detection of these diseases becomes possible.

In order to realize such a test at home, it is required that the test can be carried out with simple operations and that a small device is used for the test, and there have been attempts to develop devices for such point-of-care-testing (POCT). For example, a microtube-type device is disclosed as an analysis device that utilizes nucleic acid amplification (Patent Literature 1). This analysis device uses the so-called Eppendorf tube. A reaction is caused in the tube, the tube is irradiated with excitation light from above, and fluorescence in the tube is measured also from above. However, when the Eppendorf tube is used, it is necessary to use a heat block, and therefore, the analysis device is large in size.

### Citation List

### Patent Literature

JP 2003-190772 A

However, since a pretreatment of a sample, mixing of the sample with a nucleic acid amplification reagent, a nucleic acid amplification reaction, and detection of the reaction all need to be performed, a compact device that can be used at an individual level with simple operations has not yet been provided.

### SUMMARY OF INVENTION

### Technical Problem

With the foregoing in mind, it is an object of the present invention to provide, for example, a tool that can analyze a target in a sample with simple operations and can be downsized, and an analysis method using the same.

### Solution to Problem

In order to achieve the above object, the present invention provides an analysis cell including: a main substrate; a sample inlet cover member; and a gas outlet cover member, wherein the main substrate includes a flow path, an inlet for a sample, and a gas outlet, and the inlet and the gas outlet communicate with an outside, the inlet communicates with an upstream end portion of the flow path and the gas outlet communicates with a downstream end portion of the flow path, the flow path has a shape that expands from an upstream side toward a downstream side of the flow path, the sample inlet cover member is a liquid-tight member and can be fixed to the inlet when the sample inlet cover member is in use, and the gas outlet cover member is a liquid-tight and gas-permeable member and can be fixed to the gas outlet when the gas outlet cover member is in use.

The present invention also provides an analysis device for the analysis cell according to the present invention, including: an insertion section to which the analysis cell is to be inserted; a heating section configured to heat the analysis cell; a light source configured to irradiate the analysis cell with light; a photodetection section configured to detect light from the analysis cell; and a signal conversion section for converting the detected light to a signal.

The present invention also provides an analysis apparatus including: the analysis cell according to the present invention; and the analysis device according to the present invention.

The present invention also provides an analysis kit including the analysis device according to the present invention and the analysis cell according to the present invention.

The present invention also provides an analysis method including: an analysis unit; a storage unit; and a display unit, wherein the analysis unit is the analysis apparatus according to the present invention for analyzing a sample, the storage unit is a unit configured to store an analysis result obtained by the analysis unit, and the display unit is a unit configured to display the analytical result.

### Advantageous Effects of Invention

The present invention allows downsizing of an analysis cell, an analysis device, and the like, and also allows a target in a sample to be analyzed with simple manipulations, for example.

### BRIEF DESCRIPTION OF DRAWINGS

FIGs. 1A to 1C schematically show an example of the analysis cell of the present invention. FIG. 1A is a top view, FIG. 1B is a sectional view viewed in the arrow direction of line I-I in FIG. 1A, and FIG. 1C is a sectional view viewed in the arrow direction of line II-II in FIG. 1A.
FIGs. 2A and 2B are top views showing variations of the gas outlet in the analysis cell of the present invention.
FIG. 3 is a schematic view showing an expanding angle of the flow path in the analysis cell of the present invention.
FIGs. 4A to 4D are schematic views illustrating a method of using the analysis cell of the present invention.
FIGs. 5A and 5B are sectional views showing an example of the reagent section in the analysis cell of the present invention.
FIGs. 6A and 6B are sectional views showing another example of the reagent section in the analysis cell of the present invention.
FIG. 7 is a sectional view showing an example of the analysis cell of the present invention.
FIG. 8 is a sectional view showing another example of the analysis cell of the present invention.
FIGs. 9A to 9C are sectional views showing still another example of the analysis cell of the present invention.
FIGs. 10A and 10B are top views showing variations of the gas outlet in the analysis cell of the present invention.
FIG. 11 is a sectional view schematically showing an example of a method of using the analysis device of the present invention and the analysis cell of the present invention.
FIG. 12 is a schematic view showing an example of a display screen in the analysis system of the present invention.
FIG. 13 shows photographs of a flow path of a cell in Example 1 of the present invention.
FIG. 14 shows photographs of a flow path of a cell in Example 2 of the present invention.

### DESCRIPTION OF EMBODIMENTS

In the analysis cell of the present invention, for example, the main substrate has at least two gas outlets, and the two gas outlets are disposed in a direction perpendicular to a flow path direction.

In the analysis cell of the present invention, for example, the sample inlet cover member is a sealing member and is fixed to the inlet after the sample is injected into the analysis cell

In the analysis cell of the present invention, for example, the sample inlet cover member is a cap member and is attachable and detachable with respect to the inlet.

In the analysis cell of the present invention, for example, the main substrate has a tubular portion that protrudes upward on an upper surface of the main substrate, and an opening of the tubular portion is the inlet.

In the analysis cell of the present invention, for example, the sample inlet cover member is a cap member and can be fitted to the protruding portion.

In the analysis cell of the present invention, for example, the flow path has a reagent section and a reagent is disposed in the reagent section.

In the analysis cell of the present invention, for example, a heat-fusible film containing the reagent is disposed on the reagent section.

In the analysis cell of the present invention, for example, the heat-fusible film is at least one selected from the group consisting of agarose films, agar films, carrageenan films, and gelatin films.

In the analysis cell of the present invention, for example, a dry composition containing the reagent and a poorly water-soluble substance is disposed in the reagent section.

The analysis cell of the present invention is configured such that, for example, it further includes an attachable and detachable antifouling member, and the antifouling member is disposed behind the gas outlet cover member in a flow direction.

In the analysis cell of the present invention, for example, the main substrate further includes a gas release bypass, and one end of the gas release bypass communicates with the inlet and the other end of the gas release bypass communicates with the gas outlet via the gas outlet cover member.

In the analysis cell of the present invention, for example, a cross-sectional shape of the flow path in a direction perpendicular to the flow direction of the flow path is at least one selected from the group consisting of polygonal shapes, circular shapes, elliptical shapes, and semicircular shapes.

In the analysis cell of the present invention, for example, the main substrate is in a rectangular parallelepiped shape, among outer surfaces of the main substrate, any one outer surface that is parallel to the flow direction of the flow path is a heated surface to be heated, among the outer surfaces of the main substrate, at least one of the outer surfaces other than the heated surface is an irradiated surface to be irradiated with light, and among the outer surfaces of the main substrate, at least one of the outer surfaces other than the heated surface is an extraction surface from which light generated in the flow path is extracted.

In the analysis cell of the present invention, for example, on at least one surface of the main substrate, a region corresponding to the flow path is formed of a member that transmits excitation light.

In the analysis cell, for example, in the main substrate, a portion on a downstream side from a downstream end portion of the flow path is formed of a member that transmits fluorescence.

In the analysis cell of the present invention, for example, the main substrate is formed of a member that transmits light.

In the analysis cell of the present invention, for example, the member that transmits light is a transparent member.

In the analysis cell of the present invention, for example, the reagent is a nucleic acid amplification reagent.

In the analysis device of the present invention, for example, the insertion section is configured such that an insertion direction of the analysis cell is parallel to a direction in which the flow path of the analysis cell extends, among side surfaces of the insertion section, the heating section is disposed on an inside or an outside of any one side surface that is parallel to the insertion direction of the analysis cell, among the side surfaces of the insertion section, the light source is disposed on an inside of at least one of the side surfaces other than the side surface on which the heating section is disposed, and among the side surfaces of the insertion section, the photodetection section is disposed on an inside of at least one of the side surfaces other than the side surface on which the heating section is disposed and the side surface on which the light source is disposed.

In the analysis device of the present invention, for example, the photodetection section has a fluorescence filter, a lens, and a photodetector, and the fluorescence filter, the lens, and the photodetector are disposed in this order from a side closer to the side surface of the insertion section.

In the analysis device of the present invention, for example, a thermally conductive plate is disposed between the insertion section and the heating section.

The analysis device of the present invention further includes a terminal, for example.

The analysis device of the present invention is configured such that, for example, it further includes a storage unit, and the storage unit is configured to store signal data converted by the signal conversion section.

In the analysis device of the present invention, for example, the light source is a surface irradiation type light source.

In the analysis device of the present invention, for example, the light source is a light emitting diode (LED).

The analysis device of the present invention is configured such that, for example, the analysis system includes a terminal apparatus and a server, the terminal apparatus includes the analysis unit, the server includes the storage unit and the display unit, and the terminal apparatus and the server can be connected to each other via a communication line network.

The analysis device of the present invention is configured such that, for example, the analysis system includes a terminal apparatus and a server, the terminal apparatus includes the analysis unit, the analysis unit further includes a terminal, the server includes the storage unit and the display unit, and the terminal apparatus can be connected to the server via the terminal of the terminal apparatus.

In the analysis system of the present invention, for example, the terminal is an external connection terminal.

Hereinafter, the present invention will be described more specifically with reference to illustrative examples. It is to be noted, however, that the present invention is not limited by the following descriptions.

The present invention relates to an analysis cell, an analysis device, an analysis apparatus, and an analysis system. According to the present invention, analysis of a sample can be carried out by, for example, mixing the sample with a reagent to prepare a reaction system using the analysis cell of the present invention and then setting the reaction system in the analysis device of the present invention, thereby constituting the analysis apparatus and the analysis system of the present invention.

### [Analysis Cell]

The analysis cell of the present invention is, as described above, an analysis cell including: a main substrate; a sample inlet cover member; and a gas outlet cover member, wherein the main substrate includes a flow path, an inlet for a sample, and a gas outlet, and the inlet and the gas outlet communicate with an outside, the inlet communicates with an upstream end portion of the flow path and the gas outlet communicates with a downstream end portion of the flow path, the flow path has a shape that expands from an upstream side toward a downstream side of the flow path, the sample inlet cover member is a liquid-tight member and can be fixed to the inlet when the sample inlet cover member is in use, and the gas outlet cover member is a liquid-tight and gas-permeable member and can be fixed to the gas outlet when the gas outlet cover member is in use.

The analysis cell of the present invention can analyze a sample by, for example, inserting the analysis cell into the analysis device of the present invention to be described below.

The term "flow direction" as used in the present invention is a direction in which a sample supplied to the flow path flows, and for example, the inlet side is an upstream side and the gas outlet side is a downstream side. The term "vertical direction" as used in the present invention is a direction that extends vertically when the analysis cell of the present invention is used, and for example, in the state where the analysis cell is placed on a table, the side facing the table is a downward direction, and the opposite side is an upward direction. The term "width direction" as used in the present invention is, for example, a direction perpendicular to both the flow direction and the vertical direction.

### (1) Main substrate

As described above, the main substrate includes the flow path, the inlet, and the gas outlet, the inlet communicates with the upstream end portion of the flow path, and the gas outlet communicates with the downstream end portion of the flow path.

The main substrate may be composed of one substrate or two or more substrates, for example. In the latter case, the main substrate includes, for example, an upper substrate and a lower substrate, and the main substrate is a laminate of the upper substrate and the lower substrate.

The overall shape of the main substrate is not particularly limited. The main shape of the entire main substrate is, for example, a rectangular parallelepiped shape or a flat plate shape, and for example, among the length, the width, and the thickness thereof, the thickness in the vertical direction has the shortest length. The size of the main substrate is not particularly limited.

The material of the main substrate is not particularly limited, and may be a resin, glass, or the like. The resin preferably is a light-transmitting resin to be described below, for example.

### (2) Flow path

In the main substrate, the flow path is formed inside the main substrate, for example, and specifically, the flow path preferably is a hollow region formed inside the main substrate. When the main substrate includes the upper substrate and the lower substrate, the flow path is formed by, for example, overlaying the upper substrate with the lower substrate in such a manner that they face each other. As a specific example, in the case where the upper substrate has a recess on a surface thereof facing the lower substrate, when the upper substrate is overlaid on the lower substrate, a space is formed by the recess formed on the upper substrate and the lower substrate covering the recess, and this space serves as the flow path. In the case where the lower substrate has a recess on the surface thereof facing the upper substrate, when the upper substrate is overlaid on the lower substrate, a space is formed by the recess formed on the lower substrate and the upper substrate covering the recess, and this space serves as the flow path. Furthermore, in the case where the upper substrate and the lower substrate both have recesses on surfaces thereof facing each other, when the upper substrate is overlaid on the lower substrate, a space is formed by the recesses formed on both the substrates, and this space serves as the flow path.

As described above, the flow path has a shape that expands from the upstream side toward the downstream side (also referred to as "expanding shape" hereinafter). It is preferable that the expanding shape is such that, for example, the flow path expands in the width direction from the upstream side toward the downstream side. For example, the flow path may have the above-described expanding shape over the entire length from the upstream side to the downstream side or may have the above-described expanding shape in a part thereof. Specific examples of the flow path having the expanding shape include a flow path having a tapered shape and a flow path having a tear drop shape.

It is preferable that the expanding shape of the flow path satisfies, for example, the expression 1 < (b/a), where "a" is the cross-sectional area of the upstream end portion of the flow path and "b" is the cross-sectional area of the downstream end portion of the flow path.

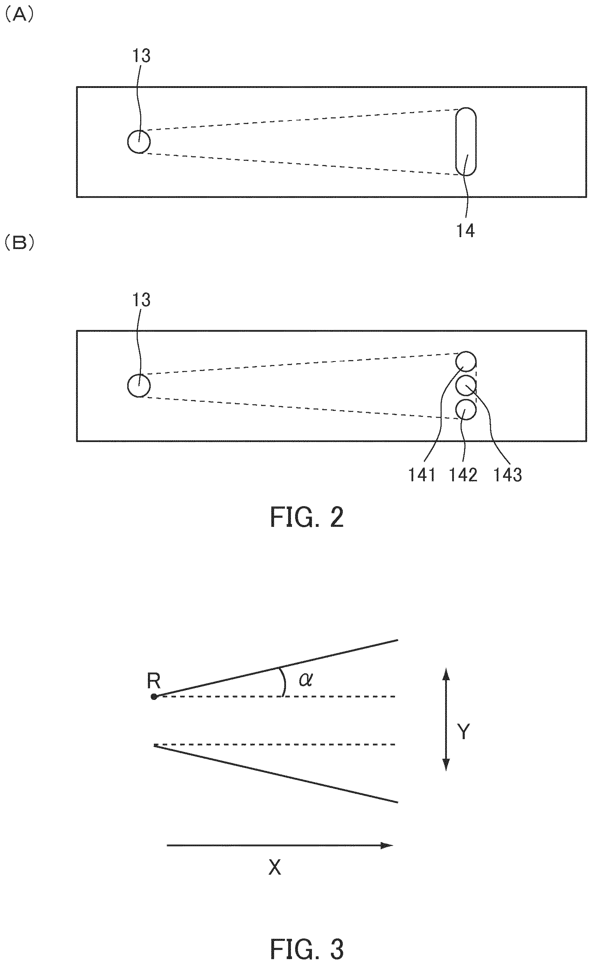

In the flow path, the expanding angle of the expanding shape is not particularly limited. The expanding angle can be expressed as, for example, an angle at which the downstream end portion expands in the width direction relative to the upstream end portion in the expanding shape of the flow path. FIG. 3 schematically shows the flow path and the expanding angle. In FIG. 3, the interior of the expanding shape is the flow path, R is the upstream end portion of the flow path, and α is the expanding angle. The expanding angle (α) is 0.01° to 60°, for example.

The cross-sectional shape of the flow path is not particularly limited. The cross section of the flow path is a cross section taken in a direction perpendicular to the flow direction of the flow path, and specifically is a cross section of a void space inside the flow path. The cross-sectional shape of the flow path may be, for example, a polygonal shape, a circular shape, or the like. The polygonal shape is, for example, a quadrangular shape, a triangular shape, an inverted triangular shape, or the like, the quadrangular shape is, for example, a square shape, a rectangular shape, a diamond shape, or the like, and the circular shape is, for example, a perfectly circular shape, an elliptical shape, a semicircular shape, or the like (the same applies hereinafter).

The inner wall of the flow path may have, for example, a flat surface, a smooth surface, or a rough surface. When the inner wall has a rough surface, the rough surface may have grooves or may have a pleated shape, for example.

In the analysis cell of the present invention, for example, it is possible to control a capillary phenomenon at a time when a sample is flowing through the flow path by the cross-sectional shape of the flow path. With this configuration, for example, it is possible to set the analysis cell of the present invention such that the same capillary phenomenon occurs throughout the entire flow path or a different capillary phenomenon occurs only at any desired region in the flow path. Accordingly, for example, when the sample is introduced to the analysis cell, it is also possible to control diffusion of a reagent in the reagent section in the flow path and the order of dissolving reagents. The capillary phenomenon may be controlled by, for example, forming both lower edges of the flow path into angular shapes or curved shapes (bent shapes), or when the flow path has a polygonal cross-section, the capillary phenomenon may be controlled by adjusting the angle of the lower edges of the flow path. When the flow path has a circular cross-section, the capillary phenomenon may be controlled by adjusting the curvature of the curved surface, for example.

In the main substrate, the number of the flow paths is not particularly limited, and the main substrate may have one flow path or two or more flow paths, for example. From the viewpoint of downsizing of the analysis cell, it is preferable that the main substrate has one flow path.

It is preferable that the width of the upstream end portion of the flow path is substantially the same as the width of the inlet, for example.

The size of the flow path is not particularly limited. It is preferable that the cross-sectional area of the hollow space of the flow path is substantially the same as the cross-sectional area of the inlet, for example. The cross-sectional area of the flow path is such that, for example, the lower limit thereof is 0.001 mm² or more, the upper limit thereof is 500 mm² or less, and the range thereof is from 0.001 mm² to 500 mm². When the flow path is in a rectangular parallelepiped shape, the width of the flow path is such that, for example, the lower limit thereof is 0.05 mm or more, the upper limit thereof is 50 mm or less, and the range thereof is from 0.05 mm to 50 mm, and the depth of the flow path is such that, for example, the lower limit thereof is 0.02 mm or more, the upper limit thereof is 10 mm or less, and the range thereof is from 0.02 mm to 10 mm.

The length of the flow path in the flow direction is such that, for example, the lower limit thereof is 1 mm or more, the upper limit thereof is 100 mm or less, and the range thereof 1 mm to 100 mm. The length of the upstream end portion in the width direction (the narrowest width) is such that, for example, the lower limit thereof is 0.05 mm or more, the upper limit thereof is 10 mm or less, and the range thereof is from 0.05 to 10 mm. The length of the downstream end portion in the width direction (the widest width) is such that, for example, the lower limit thereof is 0.05 mm or more, the upper limit thereof is 50 mm or less, and the range thereof is from 0.05 to 50 mm. The length in the vertical direction (depth) is such that, for example, the lower limit thereof is 0.02 mm or more, the upper limit thereof is 10 mm or less, and the range thereof is from 0.02 to 10 mm.

The material of the main substrate is not particularly limited. For example, in the case where the main substrate is set in an analysis device to be described below to perform optical detection, it is preferable that an irradiation region to be irradiated with light and an extraction region from which light generated by a reaction in the flow path is to be extracted are formed of light-transmitting members. Also, it is preferable that the entire main substrate is formed of a light-transmitting member, because, for example, the irradiating region and the extracting region can be set as appropriate according to the configuration of the analysis device.

The light-transmitting member is, for example, a member that does not absorb light such as excitation light and fluorescence to be detected. The member is, for example, a transparent member, and examples of the transparent member include those formed of ultraviolet-transmitting polymers such as acrylic resins, polycarbonate, polymethylpentene, and cycloolefin polymers.

### (3) Reagent section

In the analysis cell of the present invention, for example, a reagent section having a reagent disposed therein in advance may be provided in the flow path or a sample mixed with the reagent may be injected into the flow path when the analysis cell is used. In the former case, the flow path has the reagent section in a region between the upstream end portion and the downstream end portion of the flow path, for example.

When the flow path has the reagent section, the number of reagent sections is not particularly limited. For example, the flow path may have one reagent section or two or more reagent sections. In the latter case, for example, a plurality of reagents used for analysis may be disposed in the flow path as separate reagent sections. Regarding the plurality of reagents, for example, when the order of adding the reagents to the sample is set or when the reagents should not be in contact with each other until they are mixed with the sample, it is preferable that the respective reagents are disposed as separate reagent sections in the flow path. When the order of adding the plurality of reagents to the sample is set, it is preferable that the reagent sections of the respective reagents are disposed from the upstream end portion to the downstream end portion of the flow path in accordance with the order of adding the reagents, for example.

The reagent section is preferably immobilized on the flow path. The reagent section may be disposed on any of a bottom surface, an upper surface, side surfaces, and the like of the flow path, for example.

The configuration of the reagent section is not particularly limited as long as a reagent used for analysis is disposed in any desired region in the flow path directly or indirectly. It is preferable that the reagent is immobilized on the flow path, for example. Specifically, it is preferable that the reagent is immobilized on the flow path until it comes into contact with the sample and that the reagent is separated from the flow path upon contact with the sample introduced to the flow path.

Examples of the form of the reagent section include a first form in which a dry composition containing the reagent is disposed. According to this reagent section, for example, when a liquid sample comes into contact with the reagent, the reagent is dissolved in the sample. The dry composition may contain, for example, a poorly water-soluble substance in addition to the reagent. The poorly water-soluble substance is, for example, a sustained-release substance that diffuses into a liquid sample. Specific examples of the poorly water-soluble substance include hydrophilic polymers such as starch, gelatin, bovine serum albumin, cellulose, cellulose derivatives, polyacrylic acid, polyethylene oxide, polyethylene glycol, polyvinyl alcohol, and polyvinyl pyrrolidone. The liquid composition may further contain, for example, as enzyme stabilizing agents, a protecting agent such as an amino acid, a salt, or a surfactant and a sugar such as sucrose, lactose, or trehalose.

Such a reagent section can be formed by, for example, supplying the liquid composition containing the reagent to any desired region of the flow path by, for example, applying the liquid composition and then drying the liquid composition. The liquid composition contains, for example, the reagent and a solvent, and may further contain the above-described poorly water-soluble material. The solvent is not particularly limited, and may be water, a buffer solution, or the like, for example. Preferably, the poorly water-soluble substance is, for example, a substance that does not inhibit enzyme reactions, retains enzymes stably, and diffuses gradually into the sample together with the reagent upon contact with the sample.

Examples of the form of the reagent section include a second form in which a heat-fusible film containing the reagent is disposed. The term "fusible" means, for example, that a solid is mixed with another solid or liquid, and in the present invention, the term "heat-fusible film" means, for example, that the film is diffused into a sample when heated. In nucleic acid amplification, a reaction solution is heated to a reaction temperature. Primers are annealed to a template nucleic acid before the reaction solution reaches the reaction temperature, and nucleic acid amplification is caused by an enzyme at the reaction temperature. However, before the reaction solution reaches the reaction temperature, the primers may anneal non-specifically to the template nucleic acid to start a reaction. This causes non-specific amplification, resulting in measurement errors. In contrast, according to this form of the reagent section, even after a sample is introduced to the flow path, the reagent is contained in the heat-fusible film until the heat-fusible film is fused, for example. Accordingly, the reaction between the sample and the reagent is inhibited, whereby non-specific annealing and amplification are prevented. Then, when the heat-fusible film is fused by heating, the reagent is diffused into the sample, whereby a reaction between the sample and the reagent can be started.

Preferably, the heat-fusible film is a film that is fused at a temperature equal to or higher than a predetermined temperature, for example, and also can be referred to as a film that melts at a temperature equal to or higher than a predetermined temperature. The predetermined temperature is, for example, the temperature of a desired reaction by the reagent, and in the case of nucleic acid amplification, the reaction temperature is, for example, 90°C, 65°C, or 50°C. The heat-fusible film is, for example, a film formed of a heat-fusible polymer, and specific examples thereof include films formed of heat-fusible polymers such as agarose, agarose derivatives, agar, carrageenan, and gelatin. According to these heat-fusible polymers, the stability of a reagent in the film, in particular, the stability of an enzyme in a dry state, can be kept more reliably, for example. A heat-fusible film containing the reagent is obtained by, for example, mixing the reagent in a solution of the heat-fusible polymer and drying the mixture to obtain a film. The heat-fusible polymer can be selected as appropriate according to the reaction temperature in the analysis and the melting temperature of the polymer, for example.

In the present invention, the type of the reagent is not particularly limited, and can be set as appropriate according to the analysis method using the analysis cell of the present invention. As a specific example, when the analysis method is an analysis method utilizing nucleic acid amplification, the reagent is a nucleic acid amplification reagent, for example. In this case, the analysis cell of the present invention also is referred to as "nucleic acid amplification cell", for example. Examples of the nucleic acid amplification reagent include an enzyme such as a polymerase, a substrate such as dNTP, a primer, a probe, and a fluorescent substance. The respective reagents (e.g., the enzymes, primers, and the like) can be selected as appropriate according to a nucleic acid amplification method, for example. The nucleic acid amplification method is not particularly limited, and may be, for example, an isothermal amplification method or a non-isothermal amplification method (e.g., PCR or the like).

The fluorescent substance may be, for example, an intercalator such as SYBR® Green, a ruthenium complex, or the like. The fluorescent substances may also be labeling substances for probes or primers, for example.

The labeled primer may be, for example, a primer that exhibits an exciton effect, which is disclosed in Japanese Patent No. 4370385 etc. The labeled probe may be, for example, a probe that exhibits an exciton effect, which is disclosed in Japanese Patent No. 4761086 etc.

The reagent may contain, for example, a pretreatment reagent for a sample. The pretreatment reagent may be determined as appropriate depending on the type of the sample, for example.

### (4) Inlet

In the main substrate, the inlet need only be configured so as to communicate with the upstream end portion of the flow path, as described above.

The number of the inlets is not particularly limited, and for example, one flow path has one inlet. Preferably, the inlet is a through hole provided above the upstream end portion of the flow path in the main substrate, for example. The shape of the inlet is not particularly limited, and may be a polygonal shape, a circular shape, or the like.

The size of the inlet is not particularly limited. The cross-sectional area of the inlet is such that, for example, the lower limit thereof is 0.008 mm² or more, the upper limit thereof is 314 mm² or less, and the range thereof is from 0.008 to 314 mm². When the inlet has a circular shape, the radius of the cross section is such that, for example, the lower limit thereof is 0.05 mm or more, the upper limit thereof is 10 mm or less, and the range thereof is from 0.05 mm to 10 mm.

A sample is injected into the flow path from the inlet in the main substrate by introducing the tip of a sample supply tool such as a pipette tip or a dropper to the inlet, for example. Accordingly, it is preferable that, for example, a region extending from the inlet to the upstream end portion of the flow path also serves as a guide for guiding the sample supplying tool. The region extending from the opening to the upstream end portion of the flow path preferably is a hollow tubular portion, for example. The cross-sectional shape of the interior of the tubular portion is not particularly limited, and may be a polygonal shape, a circular shape, or the like. Hereinafter, the tubular portion also is referred to as "guide section".

The main substrate may have, for example, a tubular protruding portion that protrudes upward on an upper surface of the main substrate. In this case, for example, the opening of the protruding portion is the inlet, and a region extending from the inlet to the upstream end portion of the flow path is the tubular portion (guide section). The interior of the tubular portion may have a uniform size in the vertical direction, or may have a tapered shape that becomes narrower from the top toward the bottom, for example.

The length of the tubular portion in the axial direction (the height in the vertical direction) is such that, for example, the lower limit thereof is 0.1 mm or more, the upper limit thereof is 20 mm or less, and the range thereof is from 0.1 to 20 mm. When the tubular portion protrudes upward on the upper surface of the main substrate, the length of the protruding region in the axial direction is such that, for example, the lower limit thereof is 0.1 mm or more, the upper limit thereof is 20 mm or less, and the range thereof is from 0.1 mm to 20 mm.

### (5) Cover member for inlet

In the analysis cell of the present invention, the sample inlet cover member also is referred to as "inlet cover member" hereinafter. The inlet cover member is a liquid-tight member and can be fixed to the inlet when the inlet cover member is in use. In the present invention, liquid-tightness means, for example, the quality of preventing liquid from passing through. The inlet cover member need only be liquid-tight for liquid, and may be either gas-permeable or gas-tight for gas, for example. In the present invention, gas-permeability means, for example, the quality of allowing gas to pass through, and gas-tightness means, for example, the quality of preventing gas from passing through. By attaching the cover member to the inlet, it is possible to prevent the entry of external substances into the analysis cell from the inlet and to prevent a sample injected into the analysis cell from leaking outside from the inlet, for example. The cover member is fixed to the inlet after injecting a sample into the flow path of the analysis cell from the inlet, for example.

The inlet cover member may be a sealing member, for example. The sealing member is fixed to the inlet after injecting a sample into the flow path of the analysis cell, for example. The sealing member may be fixed to the inlet of the cell in such a manner that the sealing member can be detached manually after being fixed, or may be fixed firmly to the extent that manual detachment of the sealing member is difficult, for example. In the latter case, it is possible to prevent the entry of impurities from the outside and the leakage of the sample from the inside more reliably, for example.

The inlet cover member may be a cap member, for example. The cover member may be joined to the main substrate directly or indirectly, or may be a member provided independently from the main substrate and may be attached to the inlet when the inlet cover member is in use, for example.

The shape of the inlet cover member is not particularly limited, and can be set as appropriate according to the shape of the inlet. Preferably, the inlet cover member has a shape that can fit with the shape with the inlet. When the main substrate has a tubular protruding portion as described above, it is preferable that the cover member can be fitted to the protruding portion, for example. When the main substrate has the protruding portion, the cover member may be attached to the protruding portion and this portion may be used as a holding portion of the analysis cell of the present invention, for example.

From the viewpoint of handleability, the inlet cover member preferably is formed of a resin, for example. When the inlet cover member is the cap member, examples of the resin include elastomers such as TPE. When the inlet cover member is the sealing member, a heat-sealable film, an adhesive sealing member, or the like can be used, for example.

### (6) Outlet (Gas Outlet)

In the main substrate, the inlet and the gas outlet need only be configured such that, as described above, the inlet communicates with the upstream end portion of the flow path and the gas outlet communicates with the downstream end portion of the flow path.

The number of the gas outlets is not particularly limited, and for example, one flow path may have one gas outlet or may have two or more gas outlets. The gas outlet need only be disposed at a position corresponding to the downstream end portion of the flow path. When one gas outlet is provided, the position of the gas outlet in the downstream end portion is not particularly limited, and may be, for example, any position in a direction (the width direction) perpendicular to the flow path direction. As a specific example, the gas outlet may be provided at the center in the width direction or may be provided so as to extend over the entire region in the width direction, for example. When two or more gas outlets are provided, the positions of the gas outlets in the downstream end portion are not particularly limited, and may be, for example, a plurality of positions in the direction (the width direction) perpendicular to the flow path direction. As a specific example, when two gas outlets are provided, they may be provided at both ends in the width direction, and when two or more gas outlets are provided, they may be provided at both ends in the width direction and at a plurality of positions between both the ends, for example. When two or more gas outlets are provided, the positional relationship among the respective gas outlets is not particularly limited, and they may be disposed at regular intervals or irregular intervals, for example.

Preferably, the gas outlet is a through hole provided above the upstream end portion of the flow path in the main substrate, for example. The shape of the gas outlet is not particularly limited, and may be a polygonal shape, a circular shape, or the like. When one gas outlet is provided, the gas outlet may be formed in a polygonal shape (specifically, a rectangular shape), thereby providing a gas outlet in the shape of a slit that extends in the width direction, for example.

The size of the gas outlet is not particularly limited, and can be determined as appropriate according to the number of the gas outlets. When one gas outlet is provided, the cross-sectional area of the gas outlet is such that, for example, the lower limit thereof is 0.0003 mm² or more, the upper limit thereof is 31,400 mm² or less, and the range thereof is from 0.0003 to 31,400 mm². When two or more gas outlets are provided, the total cross-sectional area is in the same range as described above, for example. When the gas outlet has a circular shape, the radius of the cross section of the gas outlet is such that, for example, the lower limit thereof is 0.01 mm or more, the upper limit thereof is 10 mm or less, and the range thereof is from 0.01mm to 10 mm.

When the main substrate includes the upper substrate and the lower substrate, for example, the lower substrate may have a recess on a surface thereof facing the upper substrate, and the upper substrate may have two through holes at portions corresponding to both ends of the recess provided on the lower substrate. In this case, when the upper substrate is overlaid on the lower substrate, the recess of the lower substrate serves as the flow path, one of the through holes in the upper substrate serves as the inlet, and the other through hole serves as the gas outlet.

### (7) Cover member for gas outlet

In the analysis cell of the present invention, the cover member for the gas outlet also is referred to as "gas outlet cover member" hereinafter. The gas outlet cover member is a member that is liquid-tight and gas-permeable, and can be fixed to the gas outlet when the gas outlet cover member is in use. By attaching the cover member to the gas outlet, it is possible to ensure ventilation between the flow path and the outside by the gas outlet and to prevent a sample injected into the flow path from leaking outside from the gas outlet, for example. Furthermore, for example, since the sample does not pass through the gas outlet owing to the presence of the cover member, by setting the volume (capacity) of the flow path and introducing the sample until it reaches the gas outlet, the amount of the sample to be introduced can be made uniform among analysis cells.

As described above, the gas outlet cover member may be a member that prevents liquid from passing therethrough and allows gas to pass therethrough. Specifically, since the sample to be introduced to the analysis cell of the present invention is, for example, a sample derived from a living body, it is possible to use, as the cover member, a member that allows air to pass therethrough and does not allow so-called aqueous solvents to pass therethrough. The form of the cover member is not particularly limited, and the cover member may be a porous film or the like, for example. The cover member may be, for example, a hydrophobic film. As a specific example, a moisture-permeable waterproof film such as GORE-TEX™ can be used.

Preferably, the gas outlet cover member is fixed to the edge of the gas outlet, for example. The method for fixing the gas outlet cover member is not particularly limited, and for example, the gas outlet cover member can be fixed by means of ultrasonic welding, thermal welding, a commonly used adhesive, or the like.

### (8) Antifouling member

The analysis cell of the present invention may further include an attachable and detachable antifouling member, for example. The antifouling member is disposed behind the gas outlet cover member in the flow direction, for example, and specifically, the antifouling member is disposed so as to cover the gas outlet cover member. The antifouling member may be disposed, for example, after a sample is injected into the analysis cell, and thereafter, the analysis cell is subjected to analysis. When the analysis cell of the present invention further includes the antifouling member, it is possible to prevent the sample from leaking out of the analysis cell still more reliably.

The antifouling member is, for example, a member that is liquid-tight or gas-tight and preferably is a member that is liquid-tight and gas-tight. The antifouling member is not particularly limited, and may be a polycarbonate film, an acrylic film, or the like, for example. It is preferable that the antifouling member is fixed to the main substrate so as to cover the gas outlet cover member, for example. The antifouling member can be fixed using an adhesive, a pressure-sensitive adhesive, or the like, for example.

### (9) Gas release bypass

The analysis cell of the present invention may further include a gas release bypass, for example. For example, one end of the gas release bypass communicates with the inlet, and the other end of the gas release bypass communicates with the gas outlet via the cover member for the gas outlet. According to this configuration, out of the inlet and the gas outlet, only the inlet is an opening that communicates with the outside. Accordingly, by attaching the inlet cover member to the inlet, it is possible to prevent both the entry of substances from the outside into the analysis cell and the leakage of the sample from the inside to the outside of the analysis cell, for example.

The position of the gas release bypass in the main substrate is not particularly limited. When the analysis cell of the present invention is inserted into an analysis device to be described below to perform irradiation with excitation light and extraction of fluorescence, it is preferable that the gas release bypass is disposed in a region where the gas release bypass does not obstruct the optical paths of the excitation light and the fluorescence. As specific examples, the gas release bypass may be disposed above the flow path so as to extend in parallel to the flow path or may be disposed beside the flow path so as to extend in parallel to the flow path.

### (10) Heated surface, irradiated surface, and extraction surface

The analysis cell of the present invention can be subjected to analysis using a nucleic acid amplification reaction by inserting the analysis cell to an analysis device to be described below, for example. For an analysis using the nucleic acid amplification reaction, for example, heating is required to cause the nucleic acid amplification reaction, and in addition, irradiation with excitation light and extraction (detection) of fluorescence generated by the reaction are required for detection. In this case, it is necessary that the analysis cell of the present invention includes, for example, a heated surface to be heated, an irradiated surface to be irradiated with light, and an extraction surface from which light generated in the flow path is extracted. The respective surfaces of the analysis cell of the present invention can set according to the configuration of the analysis device to be used, for example.

As an example, when the analysis cell of the present invention is in a rectangular parallelepiped shape, the analysis cell may be configured such that, for example, among outer surfaces of the main substrate, any one outer surface that is parallel to the flow direction of the flow path is a heated surface to be heated, among the outer surfaces of the main substrate, at least one of the outer surfaces other than the heated surface is an irradiated surface to be irradiated with light, and among the outer surfaces of the main substrate, at least one of the outer surfaces other than the heated surface is an extraction surface from which light (fluorescence) generated in the flow path is extracted. Also, at least one of the outer surfaces other than the heated surface and the irradiated surface may be the extraction surface. It is to be noted, however, that the present invention is not limited to this example. For example, by using a dichroic mirror that can separate excitation light and fluorescence, the same surface can serve as the irradiated surface and the extraction surface, for example. Also, by using a transparent indium-tin-oxide heater, the same surface can serve as the heated surface and as the irradiated surface or the extraction surface for fluorescence, for example.

### (11) ID chip

The analysis cell of the present invention may further include an ID chip, for example. The ID chip may be a radio frequency identification chip (RFID) or the like, for example. According to this configuration, it is possible to manage the analysis cell of the present invention, for example.

### (12) Sample

A sample to be analyzed using the analysis cell of the present invention is not particularly limited, and examples thereof include: animal-derived samples; plant-derived samples, environmental samples such as seawater, soils, and wastewater; and food and drink samples such as drinking water and foods. Examples of the animal-derived samples include biological samples such as blood (including whole blood and separated blood cells), serum, plasma, cells (including cultured cell lines), tissues, body fluids (ear discharge, nasal discharge, pus, ascitic fluid, pleural fluid, bile, spinal fluid, sputum, etc.), mucosal cells (oral mucosal cells, gastric mucosal cells, respiratory mucosal cells, etc.), swab fluids collected from nasal mucosal membranes, oral mucosal membranes, etc. with a cotton swab or the like, sweat, amniotic fluid, excrements (urine, feces, etc.), samples obtained from respective organs by brushing using an endoscope or the like, collected fluids, biopsy samples, and alveolar lavage fluid. A sample to be introduced to the analysis cell of the present invention may be, for example, a pretreated sample or an untreated sample that has not been pretreated.

### (13) Analysis target

A target to be analyzed using the analysis cell of the present invention is not particularly limited. The analysis cell of the present invention can be used in combination with the analysis device to be described below in, for example, an analysis methods in which a heat treatment and photodetection are performed. The analysis method in which a heat treatment and photodetection are performed is, for example, a nucleic acid analysis method in which nucleic acid amplification and detection of fluorescence are performed. In this case, a nucleic acid to be analyzed as the analysis target is not particularly limited, and may be, for example, DNA, cDNA, RNA, or the like. The RNA may be mRNA, miRNA, or the like. The origin of the nucleic acid is not particularly limited, and examples thereof include viruses, bacteria, and molds. Examples of the viruses include various influenza viruses, HIV viruses, and herpes viruses. Examples of the bacteria include bacteria that cause chlamydia, *Neisseria gonorrhoeae,* and bacteria of the genus *Treponema* treponema (syphilis). Examples of the molds include fungi such as *Candida.*

The use of the analysis cell of the present invention is not limited to nucleic acid analysis. According to the analysis cell of the present invention, it is possible to measure the absorbance, fluorescence, light emission, and the like, for example. Therefore, the analysis cell of the present invention is applicable to analysis methods to be described below.

As a specific example, first, the analysis cell of the present invention can be used in an immunoassay. The immunoassay is not particularly limited, and examples thereof include an enzyme immunoassay (EIA), an enzyme-linked immunosorbent assay (ELISA), and a chemiluminescent immunoassay (CLIA). When the analysis cell of the present invention is used in an immunoassay, an antibody to a target (analyte) or an antigen is immobilized on the flow path in the analysis cell of the present invention in advance, for example. In this case, the type of the target is not particularly limited. Also, the antigen or antibody to be used is not particularly limited and can be set freely according to the type of the target. The position at which the antigen or antibody is immobilized in the flow path is not particularly limited, and the antigen or antibody may be immobilized on any of the bottom surface, upper surface, side surfaces, and the like of the flow path, for example. Then, an assay using an immunoreaction can be performed by adding the sample to the analysis cell, thereby binding the target in the sample to the antibody or antigen and further causing the target to react with the reagent in the reagent section.

As another specific example, next, the analysis cell of the present invention can be used in a biochemical analysis method. When the analysis cell of the present invention is used in a biochemical analysis method, a reagent that reacts with the target specifically is immobilized on the reagent section in the analysis cell of the present invention in advance, for example. In this case, the type of the target is not particularly limited. Also, the reagent to be used is not particularly limited, and can be set freely according to the type of the target. For example, when the blood glucose level is to be measured, the reagent may be, for example, glucose oxidase, peroxidase, 2,2' azino-bis(3-ethylbenzothiazoline 6-sulphonic acid) (ABTS), or the like. Then, analysis can be carried out by adding the sample to the analysis cell and then, for example, carrying out incubation at a temperature suitable for the reagent to cause the target to react with the reagent.

Next, the analysis cell of the present invention will be described specifically with reference to the drawings. In the respective drawings, the same components and sections are given the same reference numerals. The present invention is not limited to these illustrative examples.

### (First Embodiment)

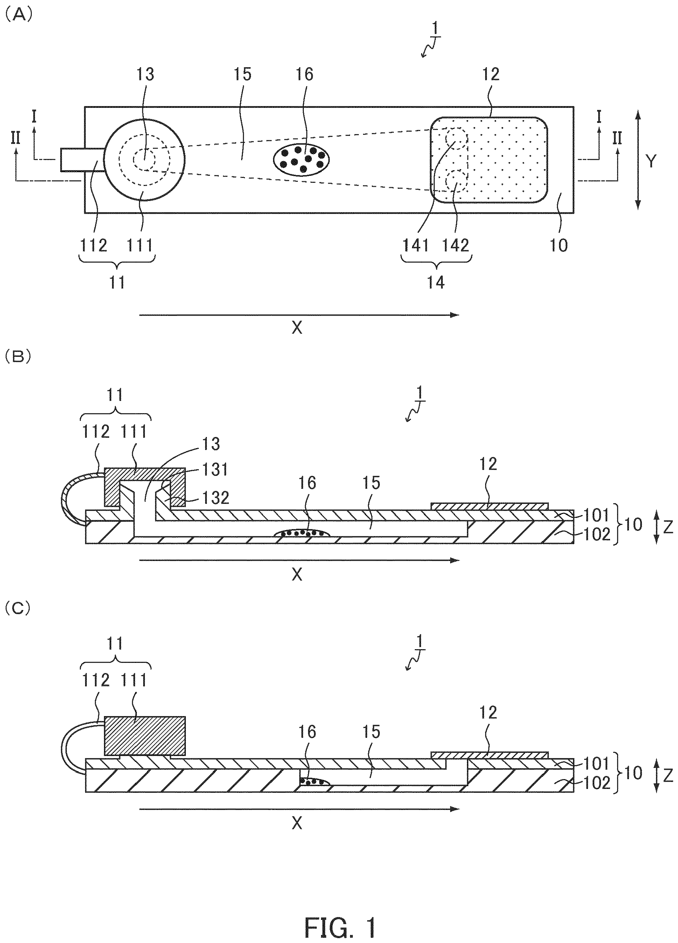

An example of an analysis cell of the present embodiment will be described with reference to FIGs. 1A to 1C and 2A and 2B.

FIGs. 1A to 1C are schematic views showing an example of the analysis cell. FIG. 1A is a top view of an analysis cell 1. FIG. 1B is a sectional view of the analysis device 1 viewed in the arrow direction of line I-I in FIG. 1A. FIG. 1C is a sectional view of the analysis device 1 viewed in the arrow direction of line II-II in FIG. 1A.

As shown in FIG. 1, the analysis cell 1 includes a main substrate 10, an inlet cover member 11, and a gas outlet cover member 12. The main substrate 10 has an inlet 13, gas outlets 14, and a flow path 15 that communicates with the inlet 13 and the gas outlets 14, and a reagent section 16 is disposed in the flow path 15. The inlet cover member 11 is joined to the main substrate 10. The inlet cover member 11 is in a cap shape and attachable and detachable with respect to the inlet 13. In FIGs. 1A to 1C, the arrow X indicates a flow direction of a sample in the flow path 15, the arrow Y indicates a width direction, and the arrow Z indicates a thickness direction.

The main substrate 10 has an upper substrate 101 and a lower substrate 102, and the upper substrate 101 is overlaid on the lower substrate 102. The upper substrate 101 and the lower substrate 102 may be integrated with each other via an adhesive, a pressure-sensitive adhesive, or the like, or may be integrated with each other by ultrasonic welding or the like, for example. The upper substrate 101 has a through hole 13 on the upstream side and two through holes 141 and 142 on the downstream side. The lower substrate 102 has a recess 15 on a surface thereof facing the upper substrate 101. In the laminate of the upper substrate 101 and the lower substrate 102, the recess 15 forms the flow path, the through hole 13 communicating with the flow path 15 on the upstream side serves as the inlet, and the through holes 141 and 142 communicating with the flow path 15 on the downstream side serve as the gas outlets 14. The flow path 15 has a shape that expands toward the direction indicated with the arrow X, and specifically, the flow path 15 has a tapered shape.

The upper substrate 101 has a tubular protruding portion 132 that protrudes upward, and the opening of the protruding portion 131 serves as the inlet 13. A region extending from the opening of the inlet 13 to the upstream end portion of the flow path 15 is a hollow tubular portion, which serves as a guide section, and a slope 131 is formed inside the inlet 13. The slope 131 allows the tip of a sample supply tool such as a dropper to be inserted into the analysis cell 1 more easily.

The inlet cover member 11 has an inlet cover member body 111 and a joint section 112, and the joint section 112 joins the inlet cover member body 1 1 1 to the main substrate 10. The joint section 112 is molded integrally with the inlet cover member main body 111 and is fixed to the main substrate 10 with an adhesive or the like, for example.

The main substrate 10 includes, on the upper surface thereof, the gas outlet cover member 12 disposed so as to cover the gas outlets 14. The gas outlet cover member 12 is fixed by means of ultrasonic welding, thermal welding, an adhesive, or the like, for example.

The shapes and sizes of the analysis cell 1 and the respective components and sections are not particularly limited, and may be as follows, for example.
Analysis cell 1
   Shape: rectangular parallelepiped shape
   Length: 45 mm
   Width: 10 mm
   Thickness: 2.6 mm
Upper substrate 101
   Shape: rectangular parallelepiped shape
   Length: 45 mm
   Width: 10 mm
   Thickness: 1.1 mm
Lower substrate 102
   Shape: rectangular parallelepiped shape
   Length: 45 mm
   Width: 10 mm
   Thickness: 1.5 mm
Flow path 15
   Interior cross-sectional shape: rectangular parallelepiped shape
   Length: 29 mm
   Width: 1.5 mm at upstream end portion
      4 mm at downstream end portion
   Depth: 0.6 mm
   Expanding angle: 5°
Inlet 13
   Shape: circular
   Inner diameter of upper end: 3 mm
   Inner diameter of tubular portion: 1.5 mm
   Height of protruding portion 132: 4 mm
Gas outlets 14 (141, 142)
   Shape: circular
   Inner diameter of upper end: 1 mm
Gas outlet cover member 12
   Shape: rectangular shape
   Length: 11 mm
   Width: 9 mm
   Thickness: 0.07 mm

It is preferable that the width of the upper end of the flow path 15 is, for example, equal to or narrower than the width of the inlet in contact with the upper end portion of the flow path 15 (the inner diameter of the tubular portion), and as a specific example, they are substantially the same. Owing to this condition and the expanding shape of the flow path 15, it is possible to sufficiently prevent air bubbles from entering the flow path 15 of the analysis cell 1, for example.

The shape and the number of the gas outlets 14 are not particularly limited as described above. FIGs. 2A and 2B are plan views showing variations of the gas outlet in the upper substrate 101. The upper substrate 101 shown in FIG. 2A has one gas outlets 14, and the shape of the gas outlets 14 is in the shape of a slit that extends along the width direction of the upper substrate 101. The upper substrate 101 shown in FIG. 2B has three gas outlets 141, 142, and 143 disposed along the width direction of the upper substrate 101.

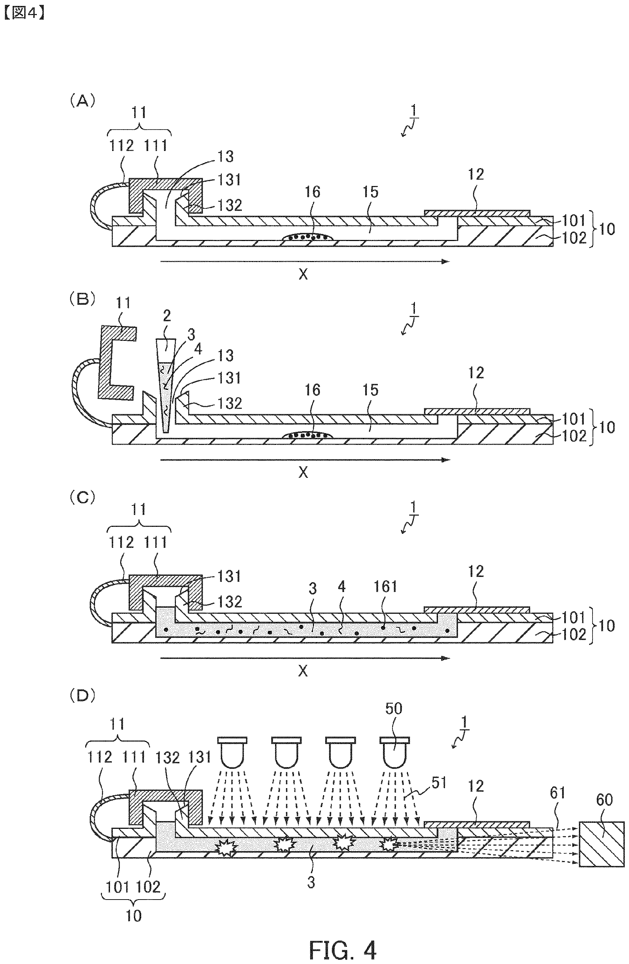

Next, a method of using the analysis cell of the present invention will be described using FIGs. 4A to 4D with reference to an example where a target in a sample is subjected to nucleic acid amplification and the obtained amplification products are detect by detecting fluorescence.

FIGs. 4A to 4D are schematic sectional views illustrating an example of an analysis cell 1 when it is used, and FIGs. 4A to 4D illustrate the respective steps. The analysis cell 1 shown in FIGs. 4A to 4D is the same as the analysis cell 1 shown in FIG. 1 except that an upper substrate 101 has the slit-shaped gas outlet 14 shown in FIG. 2A. In the present example, a reagent in a reagent section 16 is a nucleic acid amplification reagent as described above, and the nucleic acid amplification reagent contains the labeled probe that emits fluorescence. The labeled probe is a probe that emits fluorescence upon irradiation with excitation light if it is bound to a target.

As shown in FIG. 4A, in the analysis cell 1, an inlet cover member 11 is attached to an inlet 13 before the analysis cell 1 is used. The inlet cover member 11 prevents the entry of substances from the outside. First, as shown in FIG. 4B, the inlet cover member 11 is detached, and a sample 3 containing a nucleic acid is injected using a dropper 2. At this time, since the inlet 13 has a slope 131 inside, the dropper 2 can be introduced to the inlet 13 easily along the slope 131.

The sample 3 injected into a flow path 15 moves inside the flow path 15 toward the direction indicated with the arrow X by, for example, capillary action until it reaches the position where a gas outlet cover member 12 that does not allow liquid to pass therethrough is disposed. As shown in FIG. 4C, a reagent 161 (the nucleic-acid amplification reagent) in a reagent section 16 in the flow path 15 is dissolved in the sample 3 and diffuses throughout the sample 3 upon contact with the sample 3 filling the flow path 15. When the injection of the sample 3 is completed, the inlet cover member 11 is attached to the inlet 13 again.

Then, the analysis cell 1 is heated by a heating section provided outside the analysis cell 1, thereby causing a nucleic acid amplification reaction between the sample in the flow path 15 and the reagent. When a target 4 in the sample is amplified by the nucleic acid amplification reaction, the labeled probe hybridizes to the obtained amplification products. Then, as shown in FIG. 4D, the flow path 15 is irradiated with excitation light 51 emitted from light sources 50, such as LEDs, provided outside the analysis cell 1 to cause fluorescence to be generated from the labeled probe hybridized to the amplification products, and fluorescence 61 is detected by a photodetector 60. The conditions for the radiation with excitation light and the detection of the fluorescence are not particularly limited, and can be determined as appropriate according to the type of the labelling substance in the labeled probe used, for example. The above-described heating, irradiation with excitation light, and detection of fluorescence with respect to the analysis cell 1 can be performed by, for example, attaching the analysis cell 1 to an analysis device to be described below.

In the analysis cell 1, for example, the lower surface (bottom surface) is a heated surface to be heated, the upper surface is an irradiated surface to be irradiated with light, and the surface on the downstream side of the flow path 15 (the surface on the downstream side in the longitudinal direction) is an extraction surface from which light is extracted.

### (Second Embodiment)

The present embodiment relates to a reagent section in the analysis cell of the present invention.

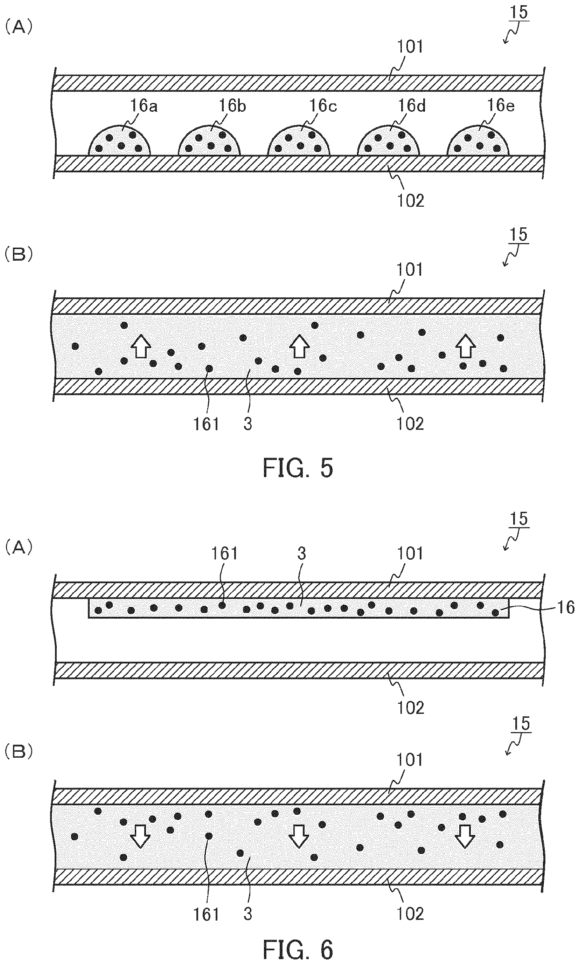

FIGs. 5A and 5B show the cross section of part of a flow path in the analysis cell of the present invention. In the present embodiment, as shown in FIGs. 5A and 5B, in a flow path 15 formed between an upper substrate 101 and a lower substrate 102, a plurality of reagent sections 16a to 16e are disposed on the lower substrate 102.

The reagent sections 16a to 16e are each formed of a dry composition containing the reagent and the poorly water-soluble substance. Each reagent section can be formed by, for example, applying a liquid composition containing the reagent, the water-insoluble substance, and the solvent to the lower substrate 102 and drying the liquid composition. In the dry composition, the concentrations of the reagent and the poorly water-soluble substance are not particularly limited, and may be set as appropriate according to the amounts of the respective reagents to be disposed in each flow path. The drying conditions are not particularly limited, and the drying may be natural drying, heat drying, air drying, freeze drying, or the like.

The reagent sections 16a to 16e may contain different reagents, respectively. The reagent sections 16a to 16e disposed in the flow path 15 preferably contain the respective reagents along the flow direction in accordance with the order of mixing the respective reagents with the sample, for example. Although the present embodiment shows an example where the reagent sections 16a to 16e are provided on the lower substrate 102, the present invention is not limited thereto, and the reagent sections 16a to 16e may be provided on the upper substrate 101. Furthermore, although the flow path 15 has a plurality of reagent sections in the present embodiment, the present invention is not limited thereto, and the flow path 15 may have only one reagent section. Also, the same reagent may be disposed in the respective reagent sections.

FIGs. 6A and 6B show the cross section of part of a flow path in the analysis cell of the present invention. In the present embodiment, as shown in FIGs. 6A and 6B, in a flow path 15 formed between an upper substrate 101 and a lower substrate 102, a reagent section 16 is disposed on the upper substrate 101.

The reagent section 16 is a heat-fusible film containing the reagent. The reagent section 16 can be formed by, for example, forming the heat-fusible polymer containing the reagent into a film. The reagent section 16 may be disposed on the upper substrate 101 by, for example, applying the heat-fusible polymer containing the reagent directly to the upper substrate 101 and then curing the heat-fusible polymer, or alternatively, forming a film of the heat-fusible polymer in advance and then disposing the thus-obtained heat-fusible film on the upper substrate 101. In the latter case, the heat-fusible film may be disposed on the upper substrate 101 by fixing it to the upper substrate 101 with an adhesive or the like, for example.

When the reagent section 16 is provided on the upper substrate 101 as described above, the heat-fusible polymer in the fused heat-fusible film diffuses downward because it has a higher specific gravity than biological samples, for example. Therefore, the reagent contained in the heat-fusible film also can be mixed with the sample efficiently while diffusing together with the heat-fusible polymer. Although the present embodiment shows an example where the reagent section 16 is provided on the upper substrate 101, the present invention is not limited thereto, and the reagent section 16 may be provided on the lower substrate 102.

### (Third Embodiment)

The present embodiment relates to the analysis cell of the present invention with an antifouling film as an antifouling member.

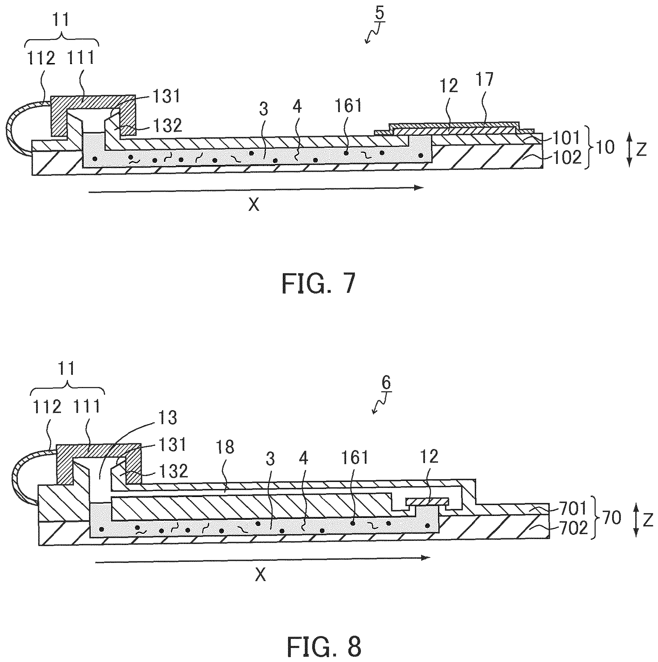

FIG. 7 shows a sectional view of an analysis cell with an antifouling film. As shown in FIG. 7, an analysis cell 5 has an antifouling film 17. The antifouling film 17 is disposed behind a liquid passage blocking member (the gas outlet cover member) 12 in the flow direction, and in FIG. 7, the antifouling film 17 is disposed on the gas outlet cover member 12 so as to cover the gas outlet cover member 12.

The antifouling film 17 can prevent a sample from leaking outside after the sample is injected and also can prevent the entry of impurities from the outside. Accordingly, the antifouling film 17 is disposed on the gas outlet cover member 12 after the sample is injected into the analysis cell 5 and before a reaction is started. It is preferable that the antifouling film 17 is in the form of a peel-off sticker in terms of ease of fixing the antifouling film 17, for example.

### (Forth Embodiment)

The present embodiment relates to an analysis cell of the present invention with a gas release bypass.

FIG. 8 shows a sectional view of an analysis cell with a gas release bypass. As shown in FIG. 8, an analysis cell 6 has a gas release bypass 18. The gas release bypass 18 is located above the flow path in the thickness direction, and one end of the gas release bypass 18 communicates with an inlet 13 and the other end of the gas release bypass 18 communicates with a gas outlet 14 via a gas outlet cover member 12.

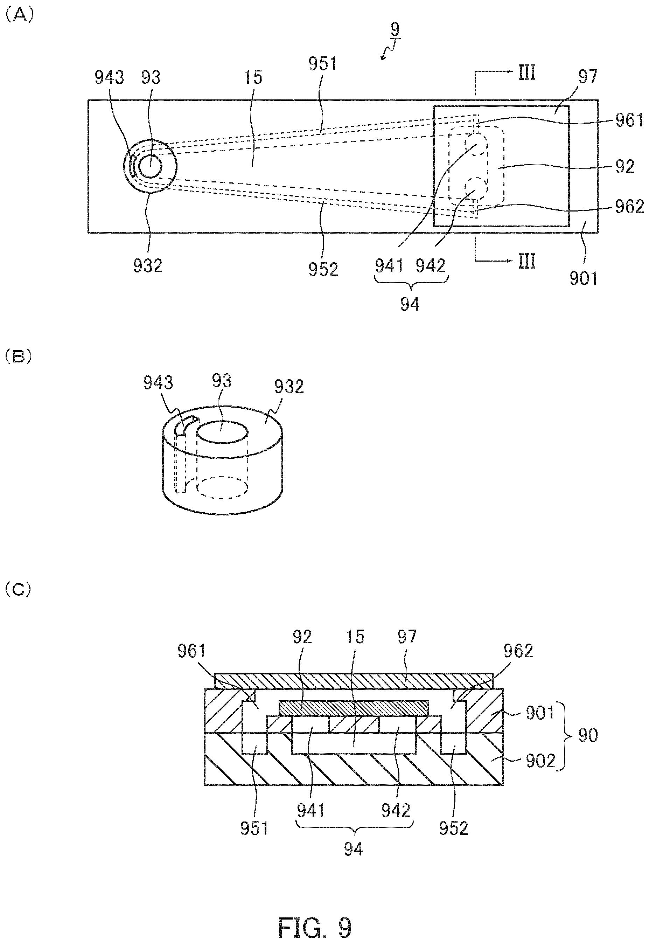

FIGs. 9A to 9C show sectional views of another analysis cell with a gas release bypass. In the analysis cell 9 shown in FIGs. 9A to 9C, the gas release bypass is provided so as to extend horizontally to a flow path in the planar direction. In FIGs. 9A to 9C, in order to explain the configuration of the gas release bypass, the inlet cover member is not shown.

FIGs. 9A to 9C are schematic views showing an example of the analysis cell. FIG. 9A is a top view of an analysis cell 9. FIG. 9B is a perspective view schematically showing a protruding portion in FIG. 9A. FIG. 9C is a sectional view of the analysis cell 9 viewed in the arrow direction of line III-III in FIG. 9A.

A main substrate 90 has an upper substrate 901 and a lower substrate 902, and the upper substrate 901 is overlaid on the lower substrate 902.

The lower substrate 902 has a recess 15 that forms a flow path, and recesses 951 and 952 that extend in the flow direction on the external sides of the recess 15 in the width direction. The recesses that extend in the flow direction serve as gas outlet paths 951 and 952, respectively.

The upper substrate 901 has a tubular protruding portion 932 that protrudes upward on the upstream side, and the opening of the protruding portion 932 serves as an inlet 93. The protruding portion 932 has a through hole 943 that penetrates a side wall portion thereof in the thickness direction. The through hole 943 is connected to the gas outlet paths 951 and 952 of the lower substrate 902 and serves as a gas outlet.

The upper substrate 901 has a recessed shape in the width direction on the downstream side, and in the thus-provided dent region (i.e., a region where the upper surface is lower than end portions in the width direction), the upper substrate 901 has two through holes 941 and 942 that serve as gas outlets. Since the gas outlets 942 and 941 are formed in the dent region, the upper substrate 901 has a space between the upper surface of the dent region and the upper surfaces of end portions in the width direction. A gas outlet cover member 92 is disposed on the upper substrate 901 so as to cover the gas outlets 942 and 941 in this space. An antifouling member 97 further is disposed on the upper substrate 901 so as to cover the dent region in the recessed shape. The antifouling member 97 in the present embodiment preferably is liquid-tight and gas-tight, for example, and may be the antifouling film shown in the above third embodiment, a sealing cap, a resin plate, or the like.

The upper substrate 901 further includes, on the external sides of the gas outlets 941 and 942 in the width direction, communication paths 961 and 962 that allow the gas outlets 941 and 942 and the gas outlet paths 951 and 952 on the lower substrate 902 to communicate with each other via the above-described space.

According to these configurations, in the analysis cell 9, which is a laminate of the upper substrate 901 and the lower substrate 902, the gas outlets 941 and 942 communicating with the flow path 15 communicate with the gas outlet paths 951 and 952 via the space and the communication paths 961 and 962. The gas outlet paths 951 and 952 communicate with the gas outlet 943 of the protruding portion 932 in the upstream end portion. On the downstream side of the analysis cell 9, the gas outlets 941 and 942 are covered with the gas outlet cover member 92, and besides, the space above the gas outlets 941 and 942 is covered with the antifouling member 97. Therefore, by providing the liquid-tight and gas-tight antifouling member 97, exhaust gas from the flow path 15 passes through the gas outlets 941 and 942 and moves inside the gas outlet paths 951 and 952 via the communication paths 961 and 962 toward the gas outlet 943 on the upstream side. To the protruding portion 932, the inlet cover member shown in FIG. 1 according to the first embodiment can be attached, for example. Accordingly, if the liquid-tight and gas-tight inlet cover member is used, by attaching the inlet cover member, both the inlet 93 and the gas outlet 943 are covered in a liquid-tight and gas-tight manner, and therefore, it is possible to prevent a sample and the exhaust gas from leaking to the outside.

### (Fifth Embodiment)

The present embodiment relates to variations of a gas outlet in the analysis cell of the present invention.

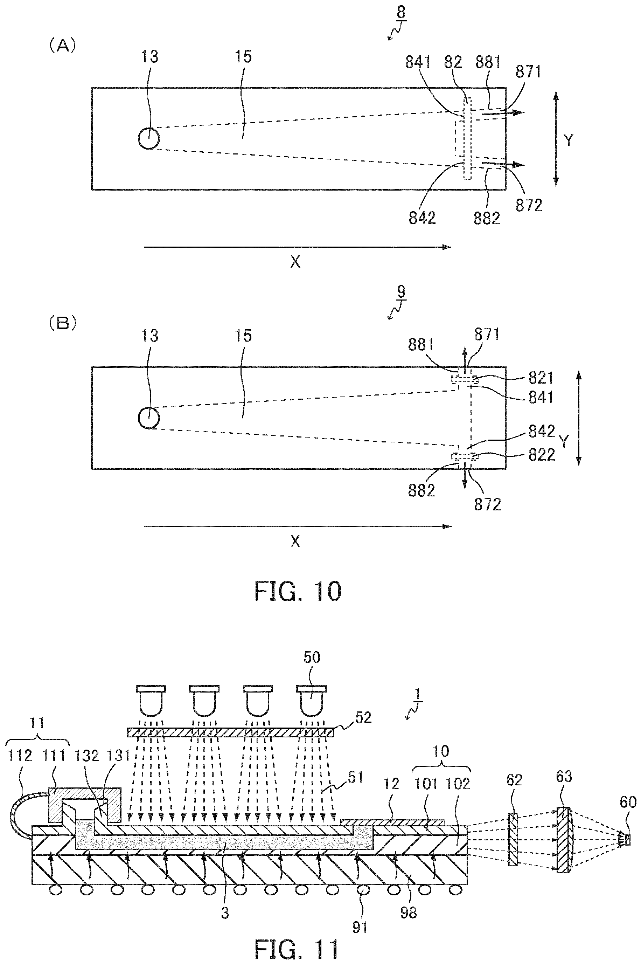

FIGs. 10A and 10B are top views each showing an analysis cell with gas outlets. Since FIGs. 10A and 10B aim to illustrate the gas outlets, other constituent elements are not shown unless otherwise stated. It is to be noted that the present invention is not limited thereto.

FIG. 10A shows an analysis cell 8 having two gas outlets on a downstream side of a flow path 15. As shown in FIG. 10A, the analysis cell 8 has two through holes 871 and 872 on a side surface in a downstream end portion, and the two through holes 871 and 872 communicate with the flow path 15 via air lead-out paths 881 and 882, respectively. In the analysis cell 8, a gas outlet cover member 82 is fixed at a position in the middle of the air lead-out paths 881 and 882 so as to extend in the cross-sectional direction. In this embodiment, regions located upstream from the gas outlet cover member 82 in the air lead-out paths 881 and 882 serve as gas outlets 841 and 842, and air that has passed through the gas outlet cover member 82 from the flow path 15 is led out to the outside from the through holes 871 and 872.

FIG. 10B shows an analysis cell 9 having two gas outlets disposed on both sides of the flow path 15 in the width direction. As shown in FIG. 10B, the analysis cell 9 has two through holes 871 and 872 on side surfaces on the downstream side, and the two through holes 871 and 872 communicate with the flow path 15 via air lead-out paths 881 and 882, respectively. In the analysis cell 9, gas outlet cover members 821 and 822 are fixed at positions in the middle of the air lead-out paths 881 and 882 so as to extend in the cross-sectional directions, respectively. In this embodiment, regions located on the flow path 15 side with respect to the gas outlet cover members 821 and 822 in the air lead-out paths 881 and 882 serve as gas outlets 841 and 842, and air that has passed through the gas outlet cover members 821 and 822 from the flow path 15 is led out to the outside from the through holes 871 and 872.

### [Analysis Device]

The analysis device of the present invention is, as described above, an analysis device for the analysis cell according to the present invention, including: an insertion section to which the analysis cell is to be inserted; a heating section configured to heat the analysis cell; a light source configured to irradiate the analysis cell with light; a photodetection section configured to detect light from the analysis cell; and a signal conversion section for converting the detected light to a signal.

In the analysis device of the present invention, the insertion section to which the analysis cell is to be inserted also may be referred to as "main body case", for example. The main body case is a housing, for example. The material thereof is not particularly limited, and the main body case may be a plastic member, for example.

In the analysis device of the present invention, the insertion section is configured such that, for example, an insertion direction of the analysis cell is parallel to a direction in which the flow path of the analysis cell extends. In this case, the analysis device may be configured such that, for example, among side surfaces of the insertion section, the heating section is disposed on an inside or an outside of any one side surface that is parallel to the insertion direction of the analysis cell, among the side surfaces of the insertion section, the light source is disposed on an inside of at least one of the side surfaces other than the side surface on which the heating section is disposed, and among the side surfaces of the insertion section, the photodetection section is disposed on an inside of at least one of the side surfaces other than the side surface on which the heating section is disposed and the side surface on which the light source is disposed. According to this configuration, for example, it is possible to separate a heating path and an excitation light incidence path for the analysis cell, whereby heating and detection of fluorescence can be achieved at the same time with high efficiency.

It is to be noted, however, that the present invention is not limited to this configuration, and for example, the surface on which the light source is disposed and the surface on which the photodetection section is disposed may be the same surface.

The heating section is not particularly limited and may be, for example, a heater. The heater preferably is a thin heater with a thickness of about 0.03 to 3 mm, for example. In the analysis device of the present invention, for example, it is preferable that a thermally conductive member is further disposed between the analysis cell to be inserted and the heating section. As the thermally conductive member, it is preferable to use, for example, an aluminum plate, a copper plate, or the like having high thermal conductivity. By using the thin heater and the thermally conductive member in combination as described above, it is possible to make the analysis device still thinner and the heating can be performed more rapidly, for example.

The light source is not particularly limited, and an LED, an optical fiber, or the like can be used, for example. The type of the light source can be determined as appropriate according to the type of a reagent in the analysis cell, for example, and it is preferable to use a light source that can emit excitation light suitable for the reagent. The analysis device of the present invention may further include, for example, an excitation light filter between the light source and the analysis cell to be inserted. The analysis device may further include, for example, a light guide plate, and the excitation light may be introduced to the inserted analysis cell using the light guide plate.

Since the analysis device of the present invention uses the cell of the present invention, it is possible to perform surface irradiation with excitation light using an LED light source, for example. For example, in conventional analyses using a micro-well plate, an Eppendorf tube, or the like, an analysis device needs to be configured such that, in order to detect amplification products that have accumulated on the bottom of the well or the tube, high-luminance excitation light is focused on the bottom of the well or the like from above the well or the like. To this end, for example, a mercury lamp, a laser, or the like is used as the light source included in the analysis device. In contrast, the cell of the present invention is a cell having a flow path, and a reaction such as nucleic acid amplification is performed in the flow path, for example. Accordingly, it is possible to perform surface irradiation with excitation light with respect to the flow path. Therefore, unlike conventional analysis devices, the analysis device of the present invention can use, for example, an LED, which has a smaller light quantity than a mercury lamp, a laser, etc., as the light source, and with the use of the LED, it is possible to achieve efficient excitation, highly sensitive detection, detection of a trace amount of a sample, and the like, for example. Also, by using an LED as the light source, the analysis device of the present inventive can be made still smaller, for example.

The photodetection section has, for example, a fluorescence filter, a lens, and a photodetector, and the fluorescence filter, the lens, and the photodetector are disposed in this order from a side closer to the side surface of the insertion section. The analysis device of the present invention may further include, for example, an optical fiber or a light guide plate, and fluorescence generated in the analysis cell may be introduced to the fluorescence filter or the lens via the optical fiber or the light guide plate, for example.

The fluorescence filter can be set as appropriate according to the type of fluorescence to be detected. The lens is a condenser lens, for example.

The photodetector may be, for example, a solid photodetector such as a charge coupled device (CCD), and specific examples thereof include photodiodes such as an avalanche photodiode (APD). Examples of the photodetector further include a photomultiplier tube.

In the analysis device, for example, an excitation filter, a dichroic mirror, or the like may be further disposed between the analysis cell to be inserted and the light source. The excitation filter and the dichroic mirror can be determined as appropriate according to the type of excitation light used for irradiation, for example. The excitation filter may be, for example, an interference filter that transmits light at specific wavelengths, and specific examples thereof include short wavelength transmission type interference filters.

The analysis device of the present invention may further include a terminal, for example. The terminal may be, for example, a USB terminal, a memory terminal, or the like, and the memory terminal encompasses a flash memory terminal and the like, for example.

Next, regarding the analysis device of the present invention having the analysis cell of the present invention inserted thereto, the relation among the respective sections will be described with reference to FIG. 11. The following example is directed to the case where a target in a sample is amplified using a nucleic acid amplification reagent and the amplification is detected by detecting fluorescence.

FIG. 11 is a schematic view showing the relation between the analysis cell 1 and the respective sections of the analysis device. Specifically, FIG. 11 shows the state where the analysis cell 1 into which a sample has been injected is inserted into the analysis device. As shown in FIG. 11, the bottom surface of the analysis cell 1 faces heating sections 91 via a thermally conductive member 98. When the analysis cell 1 is heated by the heating sections 91, a nucleic acid amplification reaction is caused between the nucleic acid amplification reagent in a flow path 15 of the analysis cell 1 and the sample. Next, the flow path 15 of the analysis cell 1 is irradiated with light emitted from light sources 50 disposed above the analysis cell 1 via an excitation light filter 52. Then, fluorescence generated in the flow path 15 of the analysis cell 1 is detected by a photodetector 60 disposed at a downstream-side terminus of the analysis cell 1 in the flow direction. At this time, between the analysis cell 1 and the photodetector 60, a fluorescence filter 62 and a lens 63 are disposed in this order from the upstream side. The fluorescence to be detected is extracted by the fluorescence filter 62, and the extracted light is concentrated by the lens 63 and then detected by the photodetector 60.

### [Analysis Apparatus]

The analysis apparatus of the present invention is, as described above, an analysis apparatus including the analysis cell according to the present invention and the analysis device according to the present invention. For the analysis apparatus of the present invention, reference can be made to the above descriptions regarding the analysis cell of the present invention and the analysis device of the present invention.

### [Analysis System]

The analysis system of the present invention is, as described above, an analysis system including: an analysis unit; a storage unit; and a display unit, wherein the analysis unit is the analysis apparatus according to the present invention for analyzing a sample, the storage unit is a unit configured to store an analysis result obtained by the analysis unit, and the display unit is a unit configured to display the analytical result.

The storage unit is a unit configured to store an analysis result obtained by the analysis apparatus according to the present invention, which is the analysis unit. The storage unit is not particularly limited, and may be, for example, a database, a server, a random access memory (RAM), a USB memory, a read-only memory (ROM), a hard disk (HD), an optical disk, a floppy disk (FD), or the like.

The display unit is a device for displaying the analytical result by the analysis unit. The display unit is not particularly limited and may be, for example, a monitor of a cellular phone, a smart phone, a tablet, a personal computer (PC), or the like.

The analysis system of the present invention may be configured such that, for example, the analysis system includes a terminal apparatus and a server, the terminal apparatus includes the analysis unit, the server includes the storage unit and the display unit, and the terminal apparatus and the server can be connected to each other via a communication line network. As described above, the analysis apparatus of the present invention can be downsized. Thus, the terminal apparatus can be a portable terminal apparatus. The terminal apparatus can be connected to the server via the communication line network, and the analytical result obtained by the terminal apparatus can be stored in the server.

The analysis system of the present invention may be configured such that, for example, the analysis system includes a terminal apparatus and a server, the terminal apparatus includes the analysis unit, the analysis unit further includes a terminal, the server includes the storage unit and the display unit, and the terminal apparatus can be connected to the server via the terminal of the terminal apparatus. As described above, the analysis apparatus of the present invention can be downsized. Thus, the analysis apparatus can be connected to the server via the terminal of the terminal apparatus, and the analytical result obtained by the terminal apparatus can be stored in the server. The terminal may be a USB terminal or the like, for example.

The analysis system of the present invention may further include, for example, in addition to the terminal apparatus for the analysis unit, a terminal apparatus for the display unit, and this terminal apparatus for display can be connected to the server via a communication line network. The terminal apparatus may be, for example, a monitor of a cellular phone, a smart phone, a tablet, a personal computer (PC), or the like.

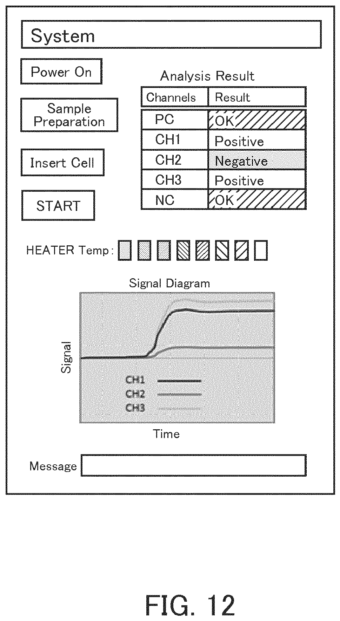

FIG. 12 shows an example of a display screen of the display unit in the analysis system of the present invention.

### Examples

### (Example 1)

A bead-containing agarose film was fixed to an upper surface of a flow path, and whether this film is fused to cause diffusion in a sample by heating was examined.

An analysis cell 1 having the same configuration as the analysis cell of the first embodiment shown in FIGs. 1A to 1C except that the reagent section 16, the cap 11, and the protruding portion 132 were not provided and that the bead-containing agarose film as shown in FIG. 6A of the second embodiment was fixed to an upper surface of a flow path 15 was produced in the following manner.

First, 3 µl of a 2% agarose solution containing 0.2% cellulose beads (particle size: 10 µm) was applied to the surface of an upper substrate 101 that forms the upper surface of the flow path 15, thereby fixing the agarose film containing the cellulose microbeads. Then, the upper substrate 101 was overlaid on a lower substrate 102, thereby producing an analysis cell 1 in which the agarose film was fixed to the upper surface of the flow path 15 as shown in FIG. 6A. As the upper substrate 101 and the lower substrate 102, UV-transparent acrylic substrates were used. The sizes and shapes of the upper substrate 101 and the lower substrate 102 were the same as those exemplified in the first embodiment, except that the upper substrate did not have the protruding portion 132 shown in FIGs. 1A to 1C. The shape and size of the flow path 15 also were the same as those exemplified in the first embodiment.

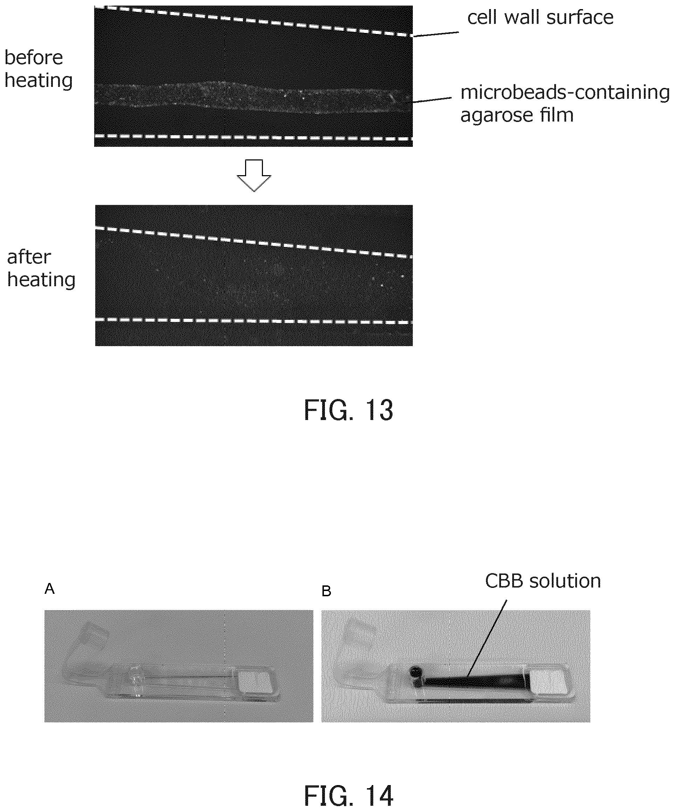

Then, the cell 1 was placed on a horizontal table with the upper substrate 101 being on the upper side and the lower substrate 102 being on the lower side. 60 µl of Milli-Q water containing 1 µg/ml of DAPI, which is a fluorescent dye, was injected into the flow path 15 of the cell 1, and thereafter, the cell 1 was heated at 65°C for 10 minutes from the upper substrate 101 side. After the heating, the cell 1 was subjected to fluorescence observation from the bottom surface side of the lower substrate 102 using an inverted fluorescence microscope. The results obtained are shown in FIG. 13. FIG. 13 shows photographs of the flow path observed from the bottom surface side of the lower substrate 102 of the cell 1. The upper photograph shows the state before the heating, and the lower photograph shows the state after the heating. As can be seen from FIG. 13, in the cell 1 before the heating, the agarose film containing the cellulose microbeads was kept fixed to the surface of the flow path 15 of the cell 1 even when the DAPI-containing water was added. In contrast, in the cell 1 after the heating, the agarose film was fused, and the microbeads were diffused into the DAPI-containing water present in the flow path 15. From this result, it was found that, by fixing an agarose film containing a reagent in a flow path, it is possible to prevent the reagent from diffusing into the flow path until the agarose film is fused. It can be said that this can prevent non-specific reactions from being caused by the reagent until the heating temperature reaches a predetermined value, for example.

### (Example 2)

An analysis cell 1 having the same configuration as the analysis cell of the first embodiment shown in FIG. 1A except that the reagent section 16, the cap 11, and the protruding portion 132 were not provided was produced. The size of a flow path 15 in the cell 1 was the same as that exemplified in the first embodiment. Then, 80 µl of a CBB solution was injected into the flow path 15 from the inlet 13 of the cell 1 using a pipette. The CBB solution was prepared by dissolving Coomassie Brilliant Blue R-250 in Milli-Q water to a final concentration of 0.1% (w/v), which is the concentration allowing easy visual observation. FIG. 14 shows photographs of the cell 1. In FIG. 14, the photograph A shows the cell 1 not filled with the CBB solution, and the photograph B shows the cell 1 filled with the CBB solution. As can be seen from FIG. 14B, the injection of the CBB solution could be achieved easily without causing any air bubbles in the cell.

Although the present invention has been described above with reference to example embodiments, the present invention is by no means limited thereto. Various changes and modifications that may become apparent to those skilled in the art may be made in the configuration and specifics of the present invention without departing from the scope of the present invention.

This application is based upon and claims the benefit of priority from Japanese Patent Application No. 2016-153132 filed on August 3, 2016, the disclosure of which is incorporated herein in its entirety by reference.

### Industrial applicability

The present invention allows downsizing of analysis cells, analysis devices, and the like, and also allows a target in a sample to be analyzed with simple manipulations, for example.

### Reference Signs List

- 1, 5, 6, 8, 9:: analysis cell
- 3:: sample
- 4:: target
- 10:: main substrate
- 101:: upper substrate
- 102:: lower substrate
- 11:: inlet cover member
- 12, 82, 821, 822:: gas outlet cover member
- 13:: inlet
- 14, 841, 842:: gas outlet
- 15:: flow path
- 16, 16a to 16e:: reagent section
- 161:: reagent
- 50:: light source
- 51:: excitation light
- 52:: excitation light filter
- 60:: photodetector
- 61:: fluorescence
- 62:: fluorescence filter
- 63:: lens
- 871, 872:: through hole
- 881, 882:: air lead-out path

## Claims

1. An analysis cell comprising:
a main substrate;
a sample inlet cover member; and
a gas outlet cover member,
wherein the main substrate comprises a flow path, an inlet for a sample, and a gas outlet, and the inlet and the gas outlet communicate with an outside,
the inlet communicates with an upstream end portion of the flow path and the gas outlet communicates with a downstream end portion of the flow path,
the flow path has a shape that expands from an upstream side toward a downstream side of the flow path,
the sample inlet cover member is a liquid-tight member and can be fixed to the inlet when the sample inlet cover member is in use, and
the gas outlet cover member is a liquid-tight and gas-permeable member and can be fixed to the gas outlet when the gas outlet cover member is in use.

2. The analysis cell according to claim 1, wherein
the main substrate has at least two gas outlets, and
the two gas outlets are disposed in a direction perpendicular to a flow direction of the flow path.

3. The analysis cell of claims 1 or 2, wherein
the sample inlet cover member is a sealing member and is fixed to the inlet after a sample is injected into the analysis cell.

4. The analysis cell according to claim 1 or 2, wherein
the sample inlet cover member is a cap member and is attachable and detachable with respect to the inlet.

5. The analysis cell according to any one of claims 1 to 4, wherein
the main substrate has a tubular protruding portion that protrudes upward on an upper surface of the main substrate, and
an opening of the protruding portion is the inlet.

6. The analysis cell according to claim 5, wherein
the sample inlet cover member is a cap member and can be fitted to the protruding portion.

7. The analysis cell according to any one of claims 1 to 6, wherein
the flow path has a reagent section and a reagent is disposed in the reagent section.

8. The analysis cell according to claim 7, wherein
a heat-fusible film containing the reagent is disposed on the reagent section.

9. The analysis cell according to claim 8, wherein
the heat-fusible film is at least one selected from the group consisting of agarose films, agar films, carrageenan films, and gelatin films.

10. The analysis cell of claim 7, wherein
a dry composition containing the reagent and a poorly water-soluble substance is disposed in the reagent section.

11. The analysis cell according to any one of claims 1 to 10, further comprising an attachable and detachable antifouling member, and
the antifouling member is disposed behind the gas outlet cover member in a flow direction.

12. The analysis cell according to any one of claims 1 to 11, wherein
the main substrate further comprises a gas release bypass, and
one end of the gas release bypass communicates with the inlet and the other end of the gas release bypass communicates with the gas outlet via the gas outlet cover member.

13. The analysis cell according to any one of claims 1 to 12, wherein
a cross-sectional shape of the flow path in a direction perpendicular to a flow direction of the flow path is at least one selected from the group consisting of polygonal shapes, circular shapes, elliptical shapes, and semicircular shapes.

14. The analysis cell according to any one of claims 1 to 13, wherein
the main substrate is in a rectangular parallelepiped shape,
among outer surfaces of the main substrate, any one outer surface that is parallel to the flow direction of the flow path is a heated surface to be heated,
among the outer surfaces of the main substrate, at least one of the outer surfaces other than the heated surface is an irradiated surface to be irradiated with light, and
among the outer surfaces of the main substrate, at least one of the outer surfaces other than the heated surface is an extraction surface from which light generated in the flow path is extracted.

15. The analysis cell according to any one of claims 1 to 14, wherein
on at least one surface of the main substrate, a region corresponding to the flow path is formed of a member that transmits excitation light.

16. The analysis cell according to any one of claims 1 to 15, wherein
in the main substrate, a portion on a downstream side from a downstream end portion of the flow path is formed of a member that transmits fluorescence.

17. The analysis cell according to any one of claims 1 to 16, wherein
the main substrate is formed of a member that transmits light.

18. The analysis cell according to any one of claims 15 to 17, wherein the member that transmits light is a transparent member.

19. The analysis cell according to any one of claims 1 to 18, wherein the reagent is a nucleic acid amplification reagent.

20. An analysis device for the analysis cell according to any one of claims 1 to 19, the analysis device comprising:
an insertion section to which the analysis cell is to be inserted;
a heating section configured to heat the analysis cell;
a light source configured to irradiate the analysis cell with light;
a photodetection section configured to detect light from the analysis cell; and
a signal conversion section for converting the detected light to a signal.

21. The analysis device of claim 20, wherein
the insertion section is configured such that an insertion direction of the analysis cell is parallel to a direction in which the flow path of the analysis cell extends,
among side surfaces of the insertion section, the heating section is disposed on an inside or an outside of any one side surface that is parallel to the insertion direction of the analysis cell,
among the side surfaces of the insertion section, the light source is disposed on an inside of at least one of the side surfaces other than the side surface on which the heating section is disposed, and
among the side surfaces of the insertion section, the photodetection section is disposed on an inside of at least one of the side surfaces other than the side surface on which the heating section is disposed and the side surface on which the light source is disposed.

22. The analysis device according to claim 21, wherein
the photodetection section comprises a fluorescence filter, a lens, and a photodetector, and
the fluorescence filter, the lens, and the photodetector are disposed in this order from a side closer to the side surface of the insertion section.

23. The analysis device according to claim 22, wherein
a thermally conductive plate is disposed between the insertion section and the heating section.

24. The analysis device according to any one of claims 20 to 23, further comprising a terminal.

25. The analysis device according to any one of claims 20 to 24, further comprising a storage unit, wherein
the storage unit is configured to store signal data converted by the signal conversion section.

26. The analysis device according to any one of claims 20 to 25, wherein
the light source is a surface irradiation type light source.

27. The analysis device according to any one of claims 20 to 26, wherein
the light source is a light emitting diode (LED).

28. An analysis apparatus comprising:
the analysis cell according to any one of claims 1 to 19; and
the analysis device according to any one of claims 20 to 27.

29. An analysis system comprising:
an analysis unit;
a storage unit; and
a display unit,
wherein the analysis unit is the analysis apparatus of claim 28 for analyzing a sample,
the storage unit is a unit configured to store an analysis result obtained by the analysis unit, and
the display unit is a unit configured to display the analytical result.

30. The analysis system according to claim 29, wherein
the analysis system comprises a terminal apparatus and a server,
the terminal apparatus comprises the analysis unit,
the server comprises the storage unit and the display unit, and
the terminal apparatus and the server can be connected to each other via a communication line network.

31. The analysis system according to claim 29, wherein
the analysis system comprises a terminal apparatus and a server,
the terminal apparatus comprises the analysis unit,
the analysis unit further comprises a terminal,
the server comprises the storage unit and the display unit, and
the terminal apparatus can be connected to the server via the terminal of the terminal apparatus.

32. The analysis system according to claim 31, wherein the terminal is an external connection terminal.
